# FASCICULE DE BREVET EUROPEEN

(11) **EP 3 752 248 B1**
(45) Date de publication et mention de la délivrance du brevet: **21.06.2023**
(21) Numéro de dépôt: 19704349.0
(22) Date de dépôt: 18.02.2019
(51) Int. Cl.: A61N 5/06

(54) **MODULE ET DISPOSITIF D'IRRADIATION TRANSCUTANÉE, EN PARTICULIER D'IRRADIATION TRANSCRÂNIENNE**
MODUL UND VORRICHTUNG ZUR TRANSKUTANEN BESTRAHLUNG, INSBESONDERE ZUR TRANSKRANIELLEN BESTRAHLUNG
MODULE AND DEVICE FOR TRANSCUTANEOUS IRRADIATION, IN PARTICULAR TRANSCRANIAL IRRADIATION

(30) Priorité: 16.02.2018 EP 18305162
(43) Date de publication de la demande: 23.12.2020
(73) Titulaire: REGENLIFE, 34000 Montpellier (FR)
(72) Inventeur: BLIVET, Guillaume, 34070 MONTPELLIER (FR); MOREAU, Guillaume, 34670 BAILLARGUES (FR); COCHARD, Etienne, 34670 BAILLARGUES (FR)
(74) Mandataire: Regimbeau
(86) Numéro de dépôt international: PCT/EP2019/053984
(87) Numéro de publication internationale: WO 2019/158758

(56) Documents cités:
- EP-A2- 2 269 692
- WO-A1-2008/144157
- US-A- 6 094 767
- US-A1- 2004 116 985
- US-A1- 2004 193 235
- US-A1- 2006 167 531
- US-A1- 2007 179 571
- US-A1- 2009 177 125
- US-A1- 2011 197 466
- US-A1- 2016 129 278

## Description

L'invention s'inscrit dans le domaine des guides optiques assurant la conduction et la diffusion d'un rayonnement lumineux, notamment à travers une surface.

L'invention s'inscrit plus particulièrement dans le domaine du traitement par irradiation lumineuse transcutanée.

L'irradiation transcutanée est une technique connue par laquelle un rayonnement d'ondes ou de particules est émis au contact de la peau et pénètre en profondeur.

On connaît comme application de cette technique la photobiomodulation et la thérapie laser de basse intensité (LLLT : Low Level Laser Therapy) utilisant des diodes lasers et/ou des diodes électroluminescentes (LEDs) et permettant de réparer et régénérer des tissus endommagés. Cette technique consiste à positionner une sonde sur la peau d'un patient et à réaliser l'émission photonique pendant un temps donné au niveau de la zone tissulaire endommagée. La sonde, par exemple commercialisée par la société THOR, comporte une tête d'émission placée sur la peau, une poignée de maintien de la tête d'émission et des câbles d'alimentation reliés à une unité de contrôle. La tête d'émission est maintenue en place par le praticien pendant toute la séance. Le document US2007/0179571 A1 peut être mentionné dans ce contexte. Voir aussi les documents US2011/0197466 A1 et EP2269692 A2.

Ces techniques d'irradiations transcutanées, notamment par photothérapie, s'appliquent également aux traitements neurologiques et psychiatriques. On parle alors d'irradiation transcrânienne. Dans ce cas, une sonde d'émission lumineuse allant du visible à l'infrarouge, du type de celle précédemment décrite, est positionnée et maintenue à la surface de la tête du patient par le praticien. Il est possible d'agir par cette technique sur des troubles neurologiques de façon thérapeutique pour restaurer ou améliorer les facultés neurologiques et cognitives, stopper la progression des désordres neuropsychiatriques, comme les maladies neurodégénératives de type Alzheimer, ou maintenir une qualité de vie.

Une autre application d'irradiation transcutanée est l'oxymétrie. Cette technique consiste à émettre des lumières rouge et infrarouge, et de mesurer leur absorption et/ou réflexion par le flux sanguin. Est connu dans ce cadre l'oxymétrie cérébrale transcrânienne qui assure la mesure de la saturation cérébrale en oxygène.

L'application de ces deux techniques pour lesquelles l'irradiation s'effectue à la surface de la tête du patient nécessite une diffusion optimisée du rayonnement lumineux impliquant le moins de perte lumineuse possible. Les guides optiques classiquement utilisés comportent une surface d'émission plane. Mais lorsque la diffusion est réalisée à la surface du crâne, les cheveux forment une barrière qui nuisent à la diffusion du rayonnement lumineux à travers le cuir chevelu. Une solution consiste à raser localement le patient, ce qui présente des inconvénients évidents.

L'invention vise principalement un guide optique de diffusion de rayonnement lumineux permettant de pallier les inconvénients précités.

L'invention vise également un guide optique apte à s'adapter à la configuration non plane de la surface à irradier, par exemple des zones couvertes de poils ou de cheveux d'un patient comme la surface du cuir chevelu d'un patient, ou le pubis. L'invention s'applique aussi à des zones dépourvues de poils ou de cheveux.

L'invention vise en outre un module d'irradiation transcutanée et un dispositif d'irradiation transcutané et transcrânien assurant la diffusion précise, efficace et contrôlée du rayonnement lumineux pour optimiser la diffusion des rayonnements lumineux vers une ou plusieurs zones cibles, notamment en vue de traitements neurologiques et psychiatriques. L'invention et ses modes de réalisation préférés sont définis dans le jeu de revendications annexé.

À cet effet, le guide optique de diffusion de rayonnement lumineux à travers une surface de l'invention est essentiellement caractérisé en ce qu'il comporte une base comprenant ou apte à maintenir une pluralité de tiges de diffusion dont l'extrémité inférieure de diffusion fait saillie de ladite base et est destinée à être appliquée sur ou à proximité de ladite surface, et dont l'extrémité supérieure collectrice est destinée à être située à proximité et en regard (c'est-à-dire en face) d'une source lumineuse alimentée électriquement, et en ce que la tige de diffusion est réalisée en un matériau apte à transmettre la lumière depuis son extrémité collectrice jusqu'à son extrémité de diffusion. Le module comprend un boîtier annulaire comportant une pluralité de sources lumineuses située en regard et à proximité de l'extrémité collectrice de la pluralité de tiges de diffusion, chaque tige de diffusion étant associée à une source lumineuse de la pluralité de sources lumineuses. Le guide optique est monté dans le boîtier annulaire. La pluralité de sources lumineuses comprend une diode électroluminescente émettant dans le spectre rouge, une diode électroluminescente émettant dans le spectre infrarouge et une diode laser infrarouge.

Le guide optique peut également comporter les caractéristiques optionnelles suivantes considérées isolément ou selon toutes les combinaisons techniques possibles :
- l'extrémité collectrice de la tige de diffusion comporte une lentille convergente ou divergente.
- l'extrémité inférieure de diffusion est plane.
- la base est réalisée en un matériau apte à transmettre la lumière.
- la base comporte une pluralité d'orifices traversant à travers chacun desquels est logée une tige de diffusion amovible.
- le guide optique est réalisé en une seule pièce intégrant la ou les tiges de diffusion.
- la tige de diffusion est réalisée en polyméthacrylate de méthyle (PMMA) ou en verre, ou en polycarbonate ou en copolyester transparent (PETG) ou encore tout autre matériau équivalent à haute transparence.

L'invention porte également sur un module d'irradiation transcutanée qui est essentiellement caractérisé en ce qu'il comporte un guide optique tel que précédemment défini, lequel guide est monté dans un boitier annulaire comportant au moins une source lumineuse alimentée électriquement et située en regard et à proximité de l'extrémité collectrice d'une tige de diffusion.

Le module peut également comporter les caractéristiques optionnelles suivantes considérées isolément ou selon toutes les combinaisons techniques possibles :
- le module comporte une pluralité de sources lumineuses, chacune des sources lumineuses étant située en regard et à proximité de l'extrémité inférieure d'une tige de diffusion.
- la source lumineuse comporte une diode laser infrarouge ou d'une diode électroluminescente (LEDs) émettant dans le spectre infrarouge, ou une diode électroluminescente (LEDs) émettant dans le spectre rouge.
- le module comporte une pluralité de sources lumineuses comprenant au moins une diode laser infrarouge, et/ou une diode électroluminescente émettant dans le spectre infrarouge, et/ou une diode électroluminescente émettant dans le spectre rouge et en ce que qu'il comporte une pluralité de tiges de diffusion, chaque tige de diffusion étant associée à une source lumineuse.
- la diode laser infrarouge est de type pulsé.
- la diode laser de type pulsé émet dans l'infrarouge à une longueur d'onde comprise entre 700 et 1200 nanomètres, présente une durée d'impulsion comprise entre 20 et 200 nanosecondes, un train d'impulsion compris entre 1 et 10 kHz, de préférence entre 1 et 20kHz, typiquement 15 kHz et une puissance impulsionnelle comprise entre 0,5 et 12 Watts.
- la fréquence de modulation appliquée aux diodes électroluminescentes et au laser infrarouge est comprise entre 1 et 1000 Hz.

L'invention porte en outre sur un dispositif d'irradiation transcutané et transcrânien qui est essentiellement caractérisé en ce qu'il comporte des moyens de positionnement sur la tête d'un utilisateur et au moins un anneau réalisé en un matériau élastique et/ou souple et apte à assurer la fixation par emprise élastique d'un module d'irradiation tel que précédemment décrit.

De préférence, le dispositif comporte une pluralité d'anneaux reliés entre eux par des éléments de jonction et les anneaux sont symétriquement disposés de part et d'autre d'un axe coïncidant avec l'axe médian de la tête lorsque le dispositif est en place sur la tête de l'utilisateur, les anneaux comportent des anneaux périphériques dont certains au moins ne sont pas reliés entre eux par des éléments de jonction.

L'invention porte enfin sur l'utilisation du guide optique tel que précédemment décrit pour diffuser un rayonnement lumineux à travers le cuir chevelu d'un utilisateur.

D'autres caractéristiques et avantages de l'invention ressortiront clairement de la description qui en est donnée ci-dessous, à titre indicatif et nullement limitatif, en référence aux figures annexées parmi lesquelles :
- les figures 1 et 2 sont des représentations schématiques en perspective de la base du guide optique de l'invention selon une première variante de l'invention respectivement depuis sa face interne (figure 1) située du côté des sources lumineuses et depuis sa face externe (figure 2) située du côté de la surface à irradier,
- la figure 3 est une représentation schématique en perspective du boitier annulaire du guide optique de l'invention selon la première variante destinée à entourer le guide optique pour assurer notamment sa rigidité,
- la figure 4 est une représentation schématique en perspective d'un anneau élastique assurant la solidarisation de la base du guide optique selon la première variante des figures 1 et 2 et du boitier annulaire de la figure 3,
- la figure 5 est une représentation schématique d'une tige de diffusion destinée à être montée sur la base du guide optique selon la première variante des figures 1 et 2,
- la figure 5a est une représentation schématique d'une tige de diffusion constituée de deux pièces ;
- la figure 6 est une représentation schématique en perspective de la face inférieure du cotée des sources lumineuses du guide optique de l'invention selon la première variante comportant des tiges de diffusion montées sur la base, laquelle base est solidarisée au boitier annulaire au moyen notamment de l'anneau élastique,
- la figure 7 est une représentation schématique en perspective du guide optique de l'invention de la figure 6 selon la première variante depuis sa face externe destinée à être apposée sur la surface à irradier,
- la figure 8 est une représentation schématique en coupe du guide optique de l'invention selon la première variante des figures 6 et 7,
- la figure 9 est une représentation schématique en perspective et en coupe du guide optique de l'invention selon une deuxième variante et comportant une base et une pluralité de tiges de diffusion montées dans ladite base,
- la figure 10 est une représentation schématique d'une tige de diffusion adaptée au guide optique de l'invention selon la deuxième variante de la figure 9,
- la figure 11 est une représentation schématique en perspective et en coupe d'un module de l'invention intégrant un guide optique selon une troisième variante de réalisation de l'invention,
- la figure 12 est une représentation schématique en perspective du guide optique de l'invention selon la troisième variante,
- la figure 13 est une représentation schématique en perspective du guide optique de l'invention selon la troisième variante représentée surmontée de la carte électronique qui intègre les sources lumineuses dirigés vers l'extrémité supérieure de chacune des tiges de diffusion,
- la figure 14 est une représentation de dessus de la carte électronique du module de la figure 11 illustrant la position des sources lumineuses,
- la figure 15 est une représentation en perspective éclatée du guide optique de l'invention depuis sa face externe du côté de la surface à irradier, et du manchon sur lequel le guide optique est destiné à être solidarisé,
- la figure 16 est une représentation schématique en perspective d'une partie du guide optique de l'invention selon la troisième variante illustrant une ailette formant moyen de solidarisation avec le manchon représenté sur la figure 15,
- la figure 17 est une représentation schématique de la partie cerclée XVII sur la figure 15 et illustrant l'élément de maintien sur le manchon de l'ailette de la figure 16,
- les figures 18A, 18B et 18C sont des représentations de côté des tiges de diffusion respectivement destinées à assurer la diffusion lumineuse d'une diode électroluminescente (LED) émettant dans l'infrarouge (figure 18A), d'une diode électroluminescente (LED) émettant dans le rouge (figure 18B) et d'une diode laser émettant dans l'infrarouge (figure 18C).
- la figure 19 est une représentation schématique éclatée d'un module de l'invention intégrant le guide optique de l'invention selon la troisième variante,
- la figure 20 est une représentation schématique en perspective de dessus du module de l'invention intégrant un guide optique selon une quatrième variante de réalisation de l'invention,
- la figure 21 est une représentation schématique éclatée en perspective de dessus du module de l'invention intégrant un guide optique selon une quatrième variante de réalisation de l'invention,
- la figure 22 est un schéma illustrant l'altération de la mémoire spatiale spontanée de souris auxquelles a été injecté le peptide amyloïde Aβ25-35, pour des souris rasées soumises à un traitement d'irradiation réalisé avec un module d'irradiation comportant une lentille plane autrement dit une plaque transparente, pour des souris non rasées soumises à un traitement d'irradiation réalisé avec un module d'irradiation comportant un guide optique selon l'invention, et pour des souris témoins non soumises à un traitement d'irradiation,
- la figure 23 est un schéma illustrant l'altération de la mémoire spatiale à long terme selon un premier test de souris auxquelles a été injecté le peptide amyloïde Aβ25-35, pour des souris rasées soumises à un traitement d'irradiation réalisé avec un module d'irradiation comportant une lentille plane autrement dit une plaque transparente, pour des souris non rasées soumises à un traitement d'irradiation réalisé avec un module d'irradiation comportant un guide optique selon l'invention, et pour des souris témoins non soumises à un traitement d'irradiation,
- la figure 24 est un schéma illustrant l'altération de la mémoire spatiale à long terme selon un second test de souris auxquelles a été injecté le peptide amyloïde Aβ25-35, pour des souris rasées soumises à un traitement d'irradiation réalisé avec un module d'irradiation comportant une lentille plane autrement dit une plaque transparente, pour des souris non rasées soumises à un traitement d'irradiation réalisé avec un module d'irradiation comportant un guide optique selon l'invention, et pour des souris témoins non soumises à un traitement d'irradiation,
- la figure 25 est un schéma illustrant le taux de peroxydation lipidique dans l'hippocampe de souris auxquelles a été injecté le peptide amyloïde Aβ25-35, pour des souris rasées soumises à un traitement d'irradiation réalisé avec un module d'irradiation comportant une lentille plane autrement dit une plaque transparente, pour des souris non rasées soumises à un traitement d'irradiation réalisé avec un module d'irradiation comportant un guide optique selon l'invention, et pour des souris témoins non soumises à un traitement d'irradiation,

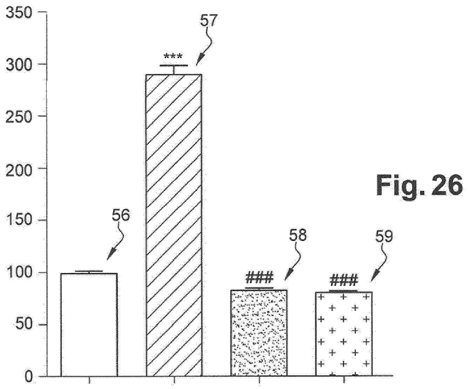

- la figure 26 est un schéma illustrant le taux de TNFα (facteur de nécrose tumorale) mesuré par la méthode immuno-enzymatique ELISA dans l'hippocampe de souris auxquelles a été injecté le peptide amyloïde Aβ25-35, pour des souris rasées soumises à un traitement d'irradiation réalisé avec un module d'irradiation comportant une lentille plane autrement dit une plaque transparente, pour des souris non rasées soumises à un traitement d'irradiation réalisé avec un module d'irradiation comportant un guide optique selon l'invention, et pour des souris témoins non soumises à un traitement d'irradiation,
- la figure 27 est une vue schématique de dessus d'un support de modules d'irradiation destiné à recevoir au moins un module de l'invention intégrant le guide optique de l'invention selon l'une ou l'autre des première, deuxième et troisième variante,
- la figure 28 est une vue schématique de côté du support de modules d'irradiation,
- la figure 29 est une vue schématique de dessus du support de modules en position sur la tête d'un utilisateur,
- la figure 30 est une vue schématique de côté du support de modules en position sur la tête d'un utilisateur,
- la figure 31 est une vue schématique de face du support de modules en position sur la tête d'un utilisateur,
- la figure 32 est une vue schématique de dos du support de modules sur la tête d'un utilisateur,
- la figure 33 est une vue schématique de côté du support de modules d'irradiation sur laquelle est également illustrée la sangle de maintien dudit support,
- la figure 34 est une vue en coupe selon la ligne XXXII-XXXII de la figure 33 d'un anneau du support de modules muni d'une rainure interne, et
- la figure 35 est une vue générale en perspective de devant du dispositif d'irradiation de l'invention comportant le support de modules d'irradiation et quatre modules en position dans quatre anneaux situés au niveau du lobe frontal.

Sur l'ensemble des figures, les éléments similaires portent des références identiques.

Le guide optique de l'invention présente une, ou de préférence plusieurs, tige(s) de diffusion lumineuse destinée(s) à être apposée(s) contre la surface à irradier. Les tiges sont avantageusement réalisées en un matériau transmettant la lumière depuis une première extrémité située à proximité d'une source lumineuse jusqu'à l'extrémité opposée située à proximité de la surface à irradier. Alternativement, les tiges peuvent comprendre un matériau transmettant la lumière depuis une première extrémité située à proximité d'une source lumineuse jusqu'à l'extrémité opposée située à proximité de la surface à irradier. Selon cette alternative, une fibre optique en matériau transmettant la lumière peut être disposée dans un conduit. Selon les première et deuxième variantes décrites ci-après, les tiges de diffusion sont des pièces indépendantes montées sur le guide optique. Selon la troisième variante, le guide optique est réalisé en une seule pièce en intégrant les tiges de diffusion. Dans les deux cas, il s'agit d'assurer la diffusion du rayonnement lumineux depuis l'extrémité de la tige du côté des sources lumineuses, jusqu'à son extrémité opposée du côté de la surface à irradier.

Selon la première variante, chaque tige est montée sur une base en formant deux pièces à part entière et en constituant ainsi le guide optique. Ainsi, le rayonnement lumineux qui pénètre au niveau de la première extrémité de la tige est intégralement conduit jusqu'à l'extrémité opposée sans risque de perte de diffusion au sein de l'épaisseur de la base.

De préférence, chaque tige comporte à son extrémité située à proximité de la surface à irradier une lentille convergente assurant la diffusion ciblée du rayonnement lumineux.

Par ailleurs, pour ne pas perdre le rayonnement lumineux qui ne pénètre pas au niveau de la première extrémité de la tige, la base est avantageusement réalisée en un matériau qui transmet également la lumière.

En outre, pour s'adapter à la surface à irradier, par exemple lorsque cette surface est le cuir chevelu, la base est réalisée en un matériau souple. Ainsi, la base peut se conformer à la configuration de la surface à irradier, tout en assurant le contact surfacique des tiges avec cette surface.

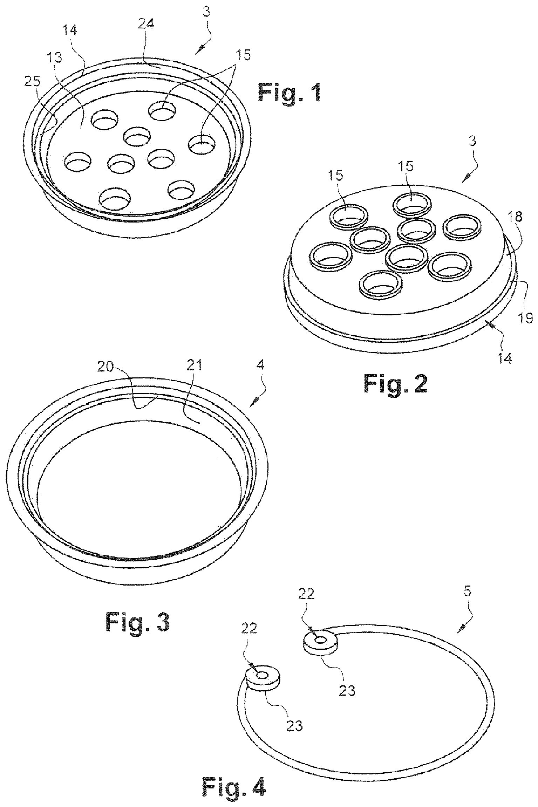

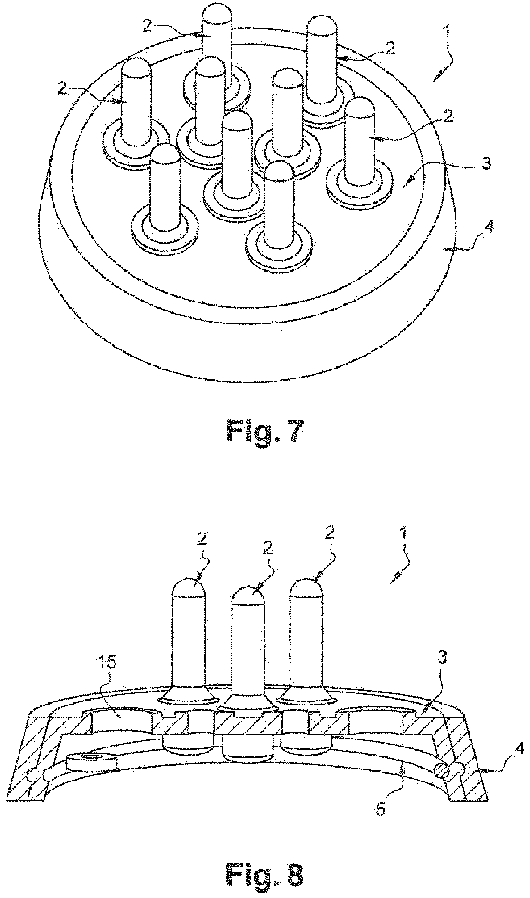

On se réfère aux figures 1 à 8 pour décrire les éléments constituant le guide optique de l'invention selon la première variante de réalisation.

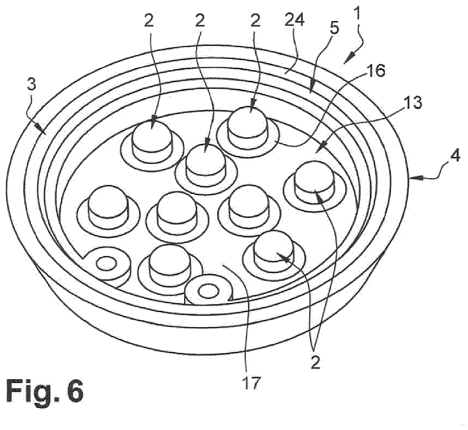

En référence aux figures 6, 7 et 8, le guide optique de l'invention 1 comporte une pluralité de tiges de diffusions 2 (dans cet exemple au nombre de neuf) montées sur une base 3, laquelle base 3 est maintenue dans un boitier annulaire 4 notamment au moyen d'un anneau élastique 5.

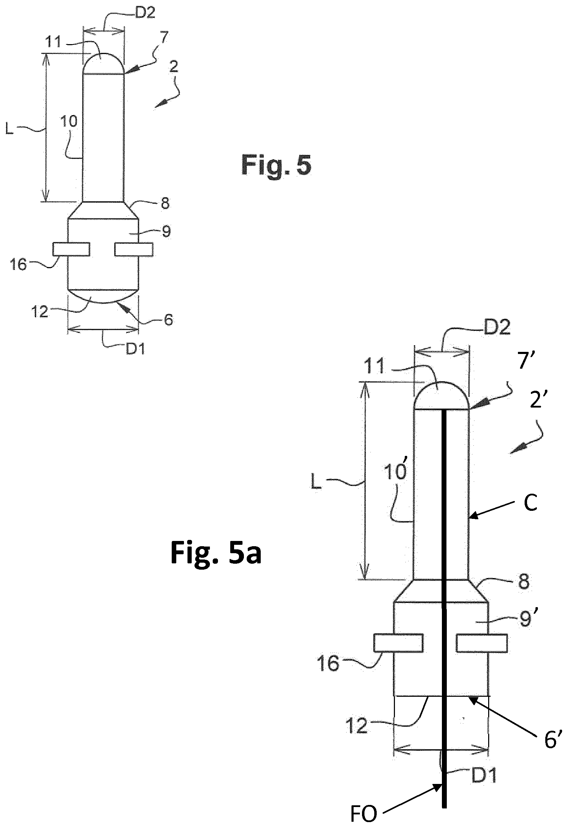

En référence à la figure 5, selon un mode réalisation, chaque tige de diffusion 2 est réalisée en une seule pièce et s'étend longitudinalement depuis une première extrémité inférieure collectrice 6 destinée à être située à proximité d'une source lumineuse, jusqu'à une extrémité opposée de diffusion 7 destinée à être apposée sur la surface à irradier, par exemple le cuir chevelu d'un utilisateur. Pour assurer au mieux la collecte du rayonnement lumineux générée par la source lumineuse (non représentée sur cette figure), le diamètre D1 de l'extrémité collectrice 6 est supérieur au diamètre D2 de l'extrémité de diffusion 7. La tige de diffusion 2 comporte longitudinalement un décroché tronconique 8 reliant la partie inférieure 9 de diamètre D1 de la tige 2 à sa partie supérieure 10 de diamètre D2. À titre d'exemple le diamètre D1 est de 2 millimètres et le diamètre D2 est de 1,5 millimètres.

La partie supérieure 10 de la tige 2 présente une longueur L comprise entre 3 et 15 millimètres, dans cet exemple de 10 millimètres. Cette longueur L doit être suffisante pour que l'extrémité de diffusion 7 soit en contact avec la surface à irradier et ce, même en présence d'une épaisseur de cheveux lorsque le guide optique est utilisé sur le cuir chevelu, mais ne doit pas être de trop grande longueur au risque d'endommager la peau.

L'optimisation de la collecte et de la diffusion du rayonnement lumineux est assurée par les paramètres indépendants suivants : la tige 2 est réalisée en un matériau qui diffuse efficacement la lumière, dans cet exemple en polyméthacrylate de méthyle (PMMA), ou en verre, ou en polycarbonate ou en copolyester transparent (PETG) ou encore en tout autre matériau équivalent à haute transparence. En outre, l'extrémité de diffusion 7 de la tige 2 comporte une lentille convergente 11 qui conduit précisément la lumière vers la cible visée. Enfin, l'extrémité collectrice 6 comporte également une lentille convergente 12 permettant de collecter efficacement le rayonnement lumineux issu de la source lumineuse correspondante qui est disposée à proximité de cette extrémité collectrice 6.

La tige de diffusion 2 est montée sur la base 3 qui est composée d'une membrane plane circulaire 13 et d'un rebord périphérique annulaire 14. La base 3 est réalisée dans cet exemple en un matériau transparent souple, par exemple en silicone. À titre d'exemple, la base 3 présente un diamètre de 40 millimètres, une hauteur de 5 millimètres et une épaisseur de 1,7 millimètres. La membrane circulaire 13 comporte des orifices traversant 15 (figures 1 et 2) à travers lesquels les tiges 2 sont insérées en force et maintenues par friction élastique. Pour renforcer le maintien des tiges 2 dans les orifices 15 et en particulier pour éviter que les tiges 2 ne s'extraient des orifices 15 du côté de la peau, la partie inférieure 9 de la tige 2 (figure 5) comporte une butée circulaire en saillie 16 qui vient en appui (figure 6) contre la face interne 17 de la base circulaire 13 de la membrane de support 3. Ainsi, les tiges 2 sont solidement maintenues sur la membrane de support 3.

En référence à la figure 2, la face externe 18 du rebord périphérique 14 de la base 3 comporte une nervure circulaire 19 destinée à se loger dans une rainure correspondante circulaire 20 ménagée sur la face interne 21 du boitier annulaire 4. Ce boitier annulaire 4 est réalisé en un matériau plus rigide que la membrane de support, par exemple en polycarbonate ou métal. Ainsi, et comme visible par exemple sur la figure 6, le boitier annulaire 4 vient entourer la plaque-support 3 au niveau de son bord périphérique 14 en conférant au guide optique 1 une rigidité permettant à ce guide 1 d'être intégré dans un module comme il sera décrit plus loin.

Pour renforcer la solidarisation de la plaque-support 3 au boitier annulaire 4, on prévoit un anneau élastique 5 (figure 4) autrement dénommé selon le terme anglo-saxon « circlip » qui présente une forme générale annulaire ouverte en comportant deux extrémités 22 situées à proximité et présentant chacune proéminence 23 qui permet de rapprocher manuellement les deux extrémités 22 jusqu'à contact pour diminuer le diamètre de l'anneau élastique 5 et permettre son insertion et son retrait.

L'anneau élastique 5 est monté au niveau de la face interne 24 du bord périphérique 14 de la base 3, et dans une rainure circulaire 25 ménagée sur cette face interne 24. L'élasticité de l'anneau 5 repousse le bord périphérique 14 de la base 3 contre le boitier circulaire 4 (figure 6) en renforçant la solidarisation de la base 3 et du boitier annulaire 4.

Le nombre et le positionnement des tiges de diffusion correspondent au nombre et au positionnement des sources lumineuses. Comme visible sur la figure 1, on prévoit dans cet exemple neuf orifices traversant 15 et donc neuf tiges de diffusion associées 2. Les neuf orifices 15 (et ainsi les neuf tiges 2) sont répartis autour du centre O de la membrane circulaire 13 à raison de trois orifices centraux et cinq orifices périphériques, tous régulièrement circulairement répartis.

Dans l'exemple décrit précédemment, le guide optique 1 est apposé sur le cuir chevelu d'un utilisateur et utilisé pour exposer certaines zones du cerveau à des rayonnements électromagnétiques allant du spectre visible à l'infrarouge. On appliquera ce type d'exposition aux traitements neurologiques et/ou psychiatriques, par exemple les pathologies neurodégénératives de type Alzheimer. On pourra également utiliser le guide optique pour réaliser des mesures d'oxymétrie cérébrale transcrânienne. Dans ce cas, certaines lentilles de diffusion 11 de chaque tige 2 joueront alors le rôle de diffuseurs de rayonnements, et d'autres lentilles de diffusion 11 de chaque tige 2 joueront le rôle de collecteurs.

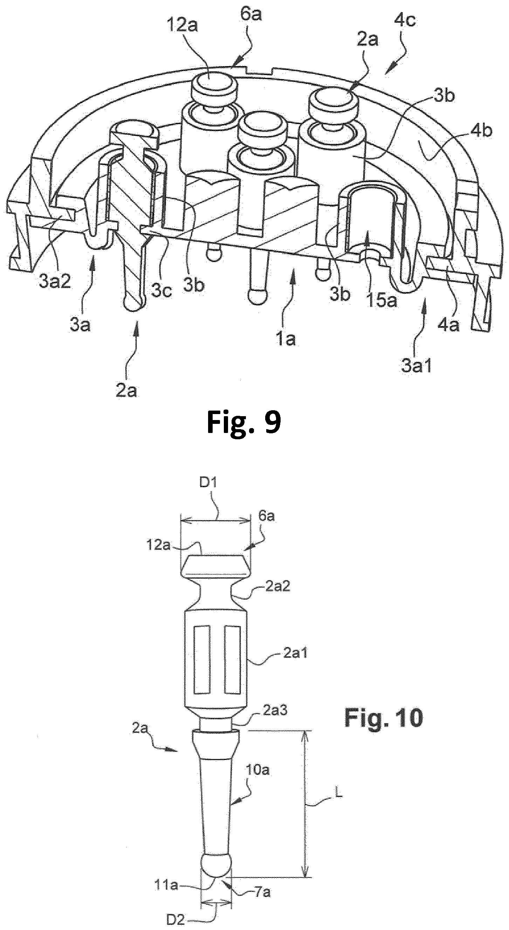

En référence aux figures 9 et 10 et selon la deuxième variante de réalisation de l'invention, la base 3a présente une forme sensiblement circulaire et comporte un bord périphérique 3a1 fait de deux portions sensiblement parallèles et délimitant une rainure 3a2 de réception d'une nervure coïncidant 4a réalisée sur la face interne annulaire 4b du boitier annulaire 4c. Ce boitier annulaire 4c diffère quelque peu structurellement du boitier annulaire 4 de la première variante de réalisation mais prétend à la même fonctionnalité de maintien du guide optique 1a dans un module d'irradiation.

Il est prévu dans cette variante que les tiges de diffusion 2a, au nombre de sept dans cet exemple, soient maintenues en position sur la base 3a au moyen de manchons 3b formant partie de la base 3 et venant enserrer la tige 2a associée lorsque cette dernière est emmanchée dans le manchon 3b. A cet effet, la base 3 est réalisée en un matériau suffisamment souple pour que les tiges e diffusion 2a puissent être emmanchées dans les manchons 3b correspondant, et le matériau constitutif de la face interne de chaque manchon 3b présente un coefficient de friction tel qu'il participe au maintien des tiges de diffusion 2a sur la base 3a. Préférentiellement, la base 3 est réalisée en une seule pièce et présente donc un coefficient de friction uniforme qui lui permet en outre de maintenir la nervure 4a du boitier annulaire 4c dans la rainure circulaire correspondante 3a2 de la base 3a.

La tige de diffusion 2a présente dans cette variante une adaptation structurelle à la présence des manchons 3b. À ce titre et plus particulièrement en référence à la figure 10, chaque tige de diffusion 2a présente une partie de fixation 2a1 essentiellement tubulaire et dont le diamètre et la longueur sont adaptés au diamètre et à la longueur des manchons 3b de la base 3a. Cette partie de fixation 2a1 est délimitée par un épaulement supérieur 2a2 du côté de l'extrémité collectrice 6a et un épaulement inférieur 2a3 du côté de l'extrémité opposée de diffusion 7a. Ce second épaulement 2a3 forme également moyen de maintien de la tige 2a sur la base 3a puisque cet épaulement 2a3 s'inscrit en coopération avec une nervure 3c (figure 9) réalisée en partie inférieure du manchon associé 3a.

On notera également que contrairement à la tige de diffusion 2 de la première variante, la tiges de diffusion 2a du guide de la deuxième variante présente à son extrémité collectrice 6a une lentille divergente 12a destinée à être située à proximité de la source lumineuse associée (non représentée) et à s'adapter au rayon de diffusion de cette source lumineuse en assurant au mieux la diffusion du rayonnement lumineux depuis l'extrémité collectrice 6a jusqu'à l'extrémité de diffusion 7 qui comporte, comme pour la première variante, une lentille convergente 11a

Comme pour la première variante, pour assurer au mieux la collecte du rayonnement lumineux générée par la source lumineuse, le diamètre D1 de l'extrémité collectrice 6a est supérieur au diamètre D2 de l'extrémité de diffusion 7a. La longueur L de la partie supérieure 10a de la tige 2a qui émerge de la base 3 en direction de la surface à irradier doit être suffisante pour que l'extrémité de diffusion 7a soit en contact avec la surface à irradier et ce, même en présence d'une épaisseur de cheveux lorsque le guide optique est utilisé sur le cuir chevelu, mais ne doit pas être de trop grande longueur au risque d'endommager la peau.

Comme pour la première variante, la tige de diffusion 2a est réalisée en un matériau qui diffuse efficacement la lumière, dans cet exemple en polyméthacrylate de méthyle (PMMA), ou en verre, ou en polycarbonate ou en copolyester transparent (PETG) ou encore en tout autre matériau équivalent à haute transparence.

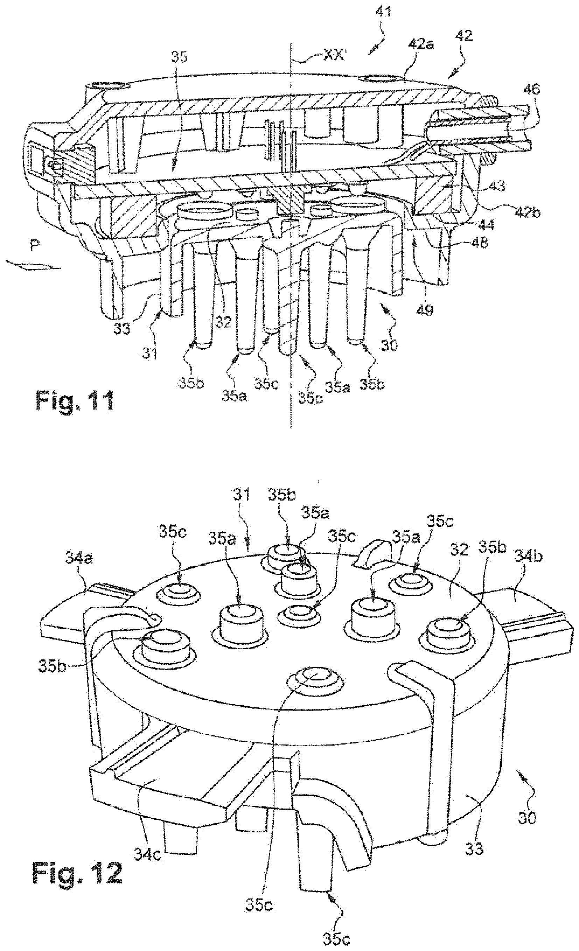

On se réfère à présent aux figures 11 à 19 pour décrire un module de l'invention intégrant la troisième variante de réalisation du guide optique de l'invention dans lequel le guide est réalisé en une seule pièce.

En référence aux figures 11 et 12, le guide optique 30 de la troisième variante comprend une base 31 faite d'une face plane circulaire 32 qui se prolonge par une jupe annulaire 33 de laquelle s'étend diamétralement en saillie vers l'extérieur trois ailettes 34a,34b,34c destinées à former moyens de solidarisation du guide optique 30 au module de l'invention 41 comme il sera décrit plus loin. Le guide optique 30 comporte dix tiges de diffusion réparties en trois groupes 35a,35b,35c selon la source lumineuse 36a,36b,36c qui leur est associée.

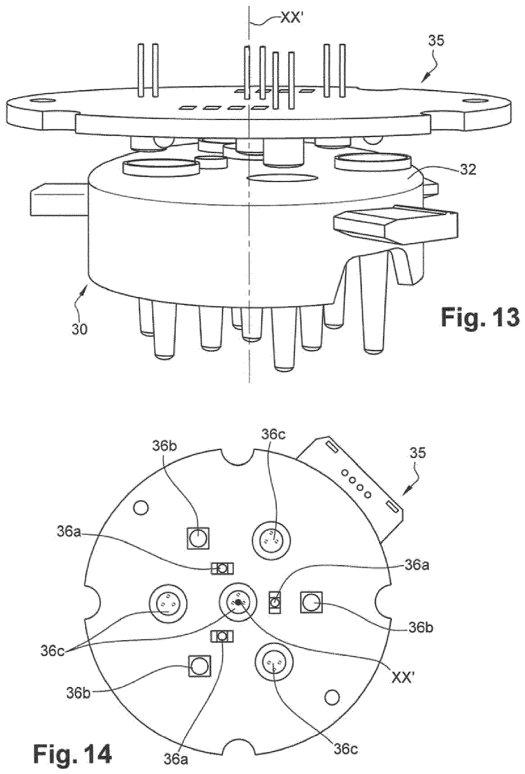

Comme illustré sur les figures 11 et 13, la face plane 32 du guide optique 30 est destinée à être agencée dans le module en regard et à proximité d'une carte électronique 35 comportant une pluralité de sources lumineuses s'étendant en saillie de la carte électronique 35 parallèlement à l'axe principal XX' de la carte électronique 35 et du module, vers le guide optique 30 et coaxialement aux tiges de diffusion associées 35a,35b,35c.

Les sources lumineuses sont également réparties en trois groupes à savoir (figure 14) :
- trois diodes électroluminescentes (LED) émettant dans l'infrarouge 36a à une longueur d'onde comprise entre 700 et 1200 nanomètres, de préférence à 850 nanomètres. Les trois LEDs infrarouge 36a sont circulairement et régulièrement réparties autour et à proximité de l'axe principal XX' de la carte électronique 35 et du module,
- trois diodes électroluminescente (LED) émettant dans le rouge 36b à une longueur d'onde comprise entre 600 et 700 nanomètres, de préférence à 625 nanomètres. Les trois LEDs rouge 36b sont circulairement et régulièrement réparties autour et à plus grand distance que les LEDs infrarouge de l'axe principal XX', et
- quatre lasers de type pulsé 36c émettant dans l'infrarouge à une longueur d'onde comprise entre 700 et 1200 nanomètres de préférence à 850 nanomètres. Chacun de ces lasers présente une durée d'impulsion comprise entre 20 et 200 nanosecondes, un train d'impulsion compris entre 1 et 10 kHz, de préférence entre 1 et 20kHz, typiquement 15 kHz et une puissance impulsionnelle comprise entre 0,5 et 12 Watts, de préférence entre 1 et 6 Watts inclus. Un premier laser 14s est centré sur l'axe XX' et les trois autres lasers sont circulairement et régulièrement répartis autour et à plus grand distance que les LEDs infrarouge de l'axe principal XX', sur la même circonférence que les LEDs rouges 36b. On peut alternativement prévoir trois lasers pulsés 14a qui seront circulairement et régulièrement répartis autour de l'axe XX'. On peut prévoir également alternativement moins de trois lasers, par exemple un unique laser pulsé centré sur l'axe XX'.

La fréquence de modulation globale appliquée aux diodes électroluminescentes et aux lasers pulsés est comprise entre 0 et 4000 Hz, de préférence comprise entre 1 et 1000 Hz, de préférence de 10 Hz.

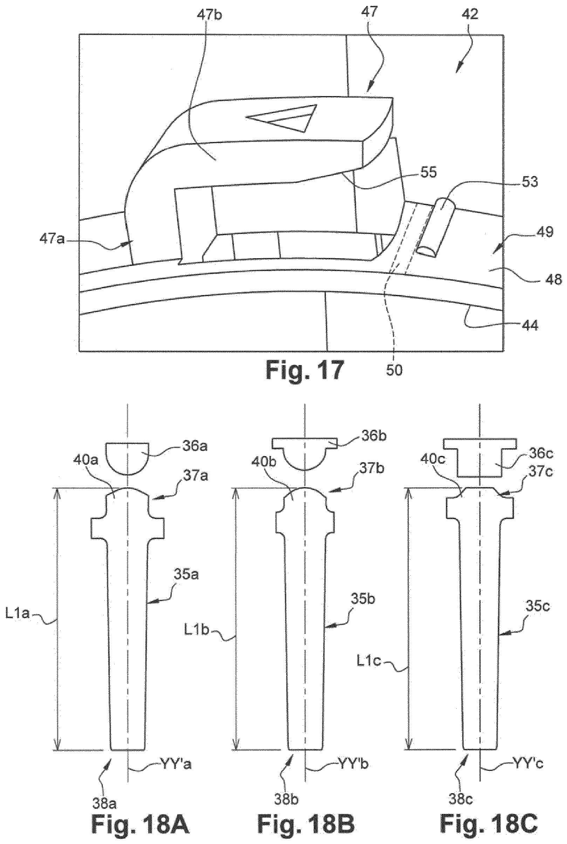

En référence aux figures 18A, 18B et 18C, chaque tige de diffusion 35a,35b,35c s'étend longitudinalement selon son axe associé YY'a, YY'b, YY'c depuis une première extrémité inférieure collectrice 37a,37b,37c située en regard proximal et coaxial de la source lumineuse associée 36a,36b,36c jusqu'à une extrémité opposée de diffusion 38a,38b,38c destinée à être apposée sur la surface à irradier, par exemple le cuir chevelu d'un utilisateur. Dans cette variante, l'extrémité de diffusion 38a,38b,38c est plane pour apporter un confort à l'utilisateur sur le cuir chevelu duquel l'extrémité de diffusion 38a,38b,38c est apposée. Chaque tige de diffusion 35a,35b,35c est de forme sensiblement conique depuis son extrémité collectrice 37a,37b,37c jusqu'à son extrémité de diffusion 38a,38b,38c.

L'optimisation de la collecte et de la diffusion du rayonnement lumineux est assurée par les paramètres indépendants suivants. Notamment, chaque tige de diffusion 35a,35b,35c est réalisée en un matériau qui diffuse efficacement la lumière, par exemple en polyméthacrylate de méthyle (PMMA), ou en verre, ou en polycarbonate ou en copolyester transparent (PETG) ou encore en tout autre matériau équivalent à haute transparence.

Concernant les émissions dans l'infrarouge et dans le rouge, les tiges de diffusion 35a,35b associées respectivement aux diodes électroluminescentes émettant dans l'infrarouge 36a et dans le rouge 36b comportent chacune une extrémité collectrice 37a,37b comprenant une lentille convergente 40a,40b qui conduit précisément la lumière en direction de l'extrémité de diffusion associée 38a,38b.

Par ailleurs, la longueur L1a,L1b des tiges de diffusion 35a,35b est ajustée à la distance focale de la lentille convergente associée 40a,40b de façon qu'au moins 40%, de préférence au moins 60%, de l'émission lumineuse totale émise par les LEDs rouge et infrarouge partant par exemple d'un angle de diffusion d'environ 120°, atteignent l'extrémité plane de diffusion correspondante 38a,38b. En référence à la figure 18c, la tige de diffusion 35c associée au laser 36c comporte une lentille plane 40c (autrement dit une plaque transparente) positionnée perpendiculairement au rayon laser dont le rayon de diffusion est confondu avec l'axe YYc'. Ce positionnement perpendiculaire assure la propagation en ligne droite du rayon laser depuis la source 36c jusqu'à l'extrémité de diffusion 38c. La distance entre l'extrémité collectrice 37c de la tige de diffusion 35c et le laser 36c est comprise entre 1 et 3 millimètres, de préférence de 2 millimètres.

A titre d'exemple et pour respecter les critères précédemment définis, chaque tige de diffusion 35a,35b,35c présente une longueur L1a,L1b,L1c comprise entre 17 et 18 millimètres, un plus grand diamètre du côté de l'extrémité collectrice 37a,37b,37c d'environ 3 millimètres, et un plus petit diamètre du côté de l'extrémité plane de diffusion 38a,38b, 38c compris entre 2,2 et 2,4 millimètres. Ainsi, toute ou majeure partie (supérieure à 40%) des rayonnements lumineux émis par les LEDs infrarouge 36a, les LEDs rouge 36b et les lasers 36c diffusent dans les tiges de diffusion 35a,35b,35c vers les extrémités collectrices associées 38a,38b,38c.

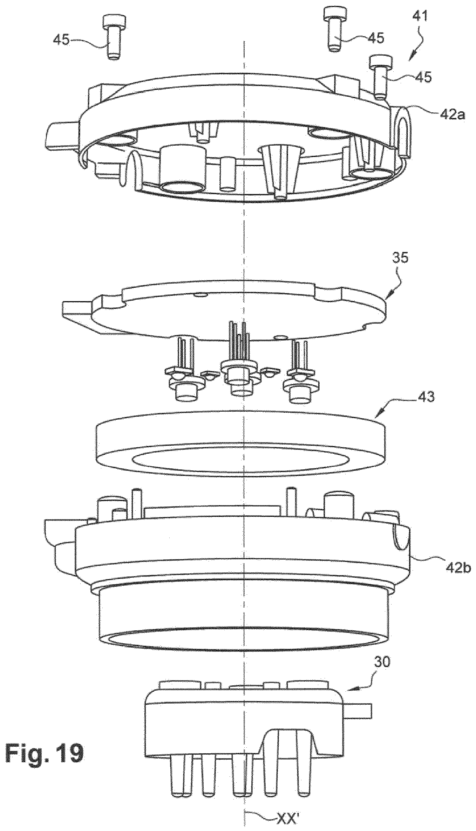

En référence aux figures 11 et 19, le guide optique 30 de l'invention est agencé dans un module 41 qui comporte un boitier annulaire 42 fait d'un capot supérieur 42a et d'un capot inférieur 42b maintenus solidaires par vissage au moyen de quatre vis 43 dont trois sont visibles sur la figure 19. Le boitier 42 assure notamment le maintien coaxial du guide optique 30 et de la carte électronique 35 comportant les sources lumineuses 36a,36b,36c. Dans cette variante, un aimant annulaire 43 est également présent entre la carte électronique 35 et le guide optique 30 et génère un champ magnétique statique compris entre 50 et 300 milliTeslas. L'aimant 43 est logé dans une rainure circulaire 44 qui est agencée sur la face supérieure d'un bord annulaire 49 du capot inférieur 42b du boitier 42 et qui entoure le guide optique 30. Ainsi, les rayonnements lumineux générées par les sources lumineuses 36a,36b,36c s'étendent perpendiculairement au plan P de l'aimant 43 et à l'intérieur de cet aimant 43. Restant dans le cadre de l'invention, le module 41 ne comporte pas d'aimant.

La carte électronique 35 présente un diamètre au moins égal au diamètre de l'aimant 43 et repose sur l'aimant 43 tout en étant solidarisé au capot supérieur 42 a du boitier 42 par un système de vissage ou encliquetage non représenté sur les figures. La carte électronique 35 est alimentée électriquement via un câble électrique 46 (figure 11) qui s'étend en dehors du module 41. Le câble électrique 46 renferme également des fils de données (par exemple de type bus CAN ou série RS485), un fil d'alimentation de la ou des sources d'irradiation, d'un ou plusieurs fils de masse et peut être blindé.

Comme visible sur la vue éclatée de la figure 19, les principaux composants du module 41 (boitier 42, carte électronique 35, aimant 43 et guide optique 30) sont coaxialement agencés autour de l'axe principale XX' du module 41.

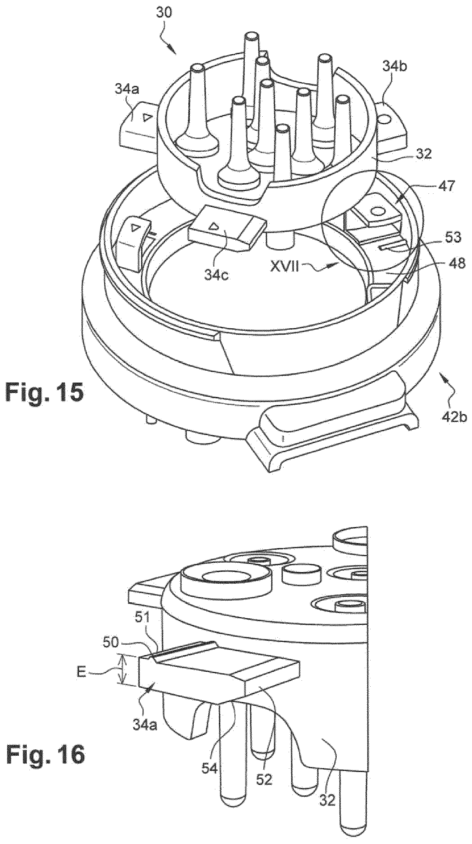

La solidarisation entre le guide optique 30 et le capot inférieur 42b du capot 42, ainsi que les moyens associés à cette solidarisation sont décrits en référence aux figures 15 à 17.

Les trois ailettes 34a,34b,34c régulièrement réparties en saillie sur la jupe 33 de la base 33 du guide optique 30 sont destinées à venir en prise dans un organe d'emprise 47 ménagé sur la face inférieure 48 du bord annulaire 49 du capot inférieure 42b du boitier 42, dont la face supérieure 44 forme rainure de réception de l'aimant 43. L'organe d'emprise 47 est en forme de L avec une première partie 47a formant butée de l'ailette correspondante 34a,34b,34c et une seconde partie 47b sensiblement perpendiculaire à la première partie 47a et s'étendant parallèlement à la face inférieure 48 du bord annulaire 49 à une distance de cette face 48 qui coïncide avec l'épaisseur E de l'ailette 34a,34b,34c pour permettre l'emprise de l'ailette 34a,34b,34c dans l'organe d'emprise 47.

En outre, comme visible sur la figure 16, chaque ailette 34a présente un bossage 50 ménagé sur toute la largeur de l'ailette 34a à proximité de son extrémité libre 51 opposée à l'extrémité libre 52 destinée à venir en butée dans l'organe d'emprise 47. Ce bossage 50 coïncide avec un autre bossage 53 ménagée sur tout ou partie de la largeur de la face inférieure 48 du bord annulaire 49 du capot inférieur 42 de sorte que lorsque l'ailette 34a est logée dans l'organe d'emprise 74, le bossage 50 de l'ailette 34a soit situé en aval du bossage 53 (comme illustré en pointillé sur la figure 17). Le maintien de l'ailette 34a dans l'organe d'emprise 47 est ainsi assuré.

Enfin, pour faciliter l'insertion de l'ailette 34a dans l'organe d'emprise 47, la face inférieure de l'ailette 34a présente un biseau 54 au niveau de l'extrémité libre 52 destinée à venir en butée dans l'organe d'emprise, qui coïncide avec un biseau 55 ménagé sur la face inférieure de la seconde partie 47b de l'organe d'emprise 47.

Le guide optique de l'invention selon la quatrième variante de l'invention présente une alternative aux moyens de solidarisation du guide optique 30 au module de l'invention 41. Ces moyens de solidarisation comportent deux pattes d'encliquetage 34a1 ménagées au niveau de la base 31 du guide optique 30 en étant diamétralement opposées et s'étendant au niveau de la jupe annulaire 33. Chaque patte d'encliquetage 34a1 coïncide avec un logement 47a1 ménagé au niveau de la face interne du capot inférieure 42b du boitier 42, lequel logement 47a1 comporte un bossage 47a2 qui empêchant le retrait du guide optique 30 du module 41 lorsque le guide optique est logé dans le boitier 42 et que par retour élastique, les pattes d'encliquetage 34a1 viennent en appui de contact contre la face interne du capot inférieur 42b. On comprend que le retrait du guide optique 30 du module 41 s'effectue par un effort concomitant exercé sur les deux pattes d'encliquetage 34a1 et dirigé vers le centre du module 41.

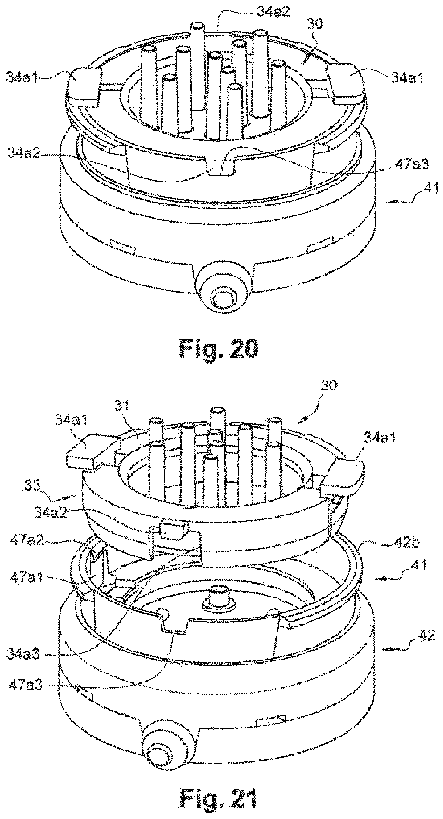

Le positionnement précis du guide optique 30 dans le module 41 ainsi que son maintien notamment en rotation relativement au boitier 42, sont améliorés par la présence de deux pattes rigides 34a2 ménagées au niveau de la base 31 du guide optique 30 en étant diamétralement opposés et positionnées à 90° par rapport aux pattes d'encliquetage 34a1. Chaque patte rigide 34a2 fait saillie, dans le plan de la jupe annulaire 33 du guide optique, au niveau d'un décroché 34a3 réalisé dans la jupe annulaire 33. Deux évidements coïncidant 47a3 sont réalisés au niveau du bord annulaire du capot inférieur 42b. Comme illustré sur la figure 20, lorsque le guide optique 30 est monté sur le boitier 42, les pattes rigides 34a2 se logent dans les évidemment 47a3.

Tous les autres éléments du guide optique 30 et du boitier 42 sont inchangés par rapport à la troisième variante des figures 11 à 19.

### Tests et protocoles

Des tests ont été menés pour évaluer l'efficacité du dispositif de l'invention. Il a été plus précisément été évalué l'efficacité du dispositif de l'invention sur l'atténuation de la pathologie induite par l'injection de bêta-amyloïde chez des souris. Ces tests ont également permis de déterminer le protocole d'irradiation permettant d'agir sur les maladies neurodégénératives telles que la maladie d'Alzheimer.

Le modèle animal utilisé pour tester le dispositif de l'invention est le modèle non transgénique Aβ25-35 de la maladie d'Alzheimer consistant en l'injection intracérébro-ventriculaire chez la souris du peptide amyloïde Aβ25-35 sous forme oligomèrique. La présence de peptide amyloides a été identifiée dans le cerveau de malades Alzheimer ; le peptide Aβ25-35 se trouve être l'un des plus neurotoxiques. Il a été montré que l'injection intracérébro-ventriculaire du peptide Aβ25-35 résulte sept jours après au niveau du cerveau dans la présence d'une neuroinflammation et gliose réactive, l'activation de caspases pro-apoptotiques, un stress oxydant, une réduction du nombre de cellules pyramidales dans l'hippocampe, une perte de neurones cholinergiques et de sérieux troubles de mémoire. De manière très intéressante, l'injection du peptide Aβ25-35 résulte dans l'installation d'une pathologie qui présente toutes les caractéristiques de la maladie d'Alzheimer chez l'homme avec en particulier l'accumulation d'espèces Aβ endogènes mais aussi une hyperphosphorylation de la protéine tau, comme observé dans la physiopathologie de la maladie d'Alzheimer.

Au jour 1, on a injecté à un groupe de souris le peptide amyloïde Aβ25-35 à raison de 9 nmol/souris et à un autre groupe de souris contrôles le peptide Sc.Aβ (scrambled amyloid-β protein 25-35) à raison également de 9 nmol/souris afin de provoquer une toxicité amyloïde.

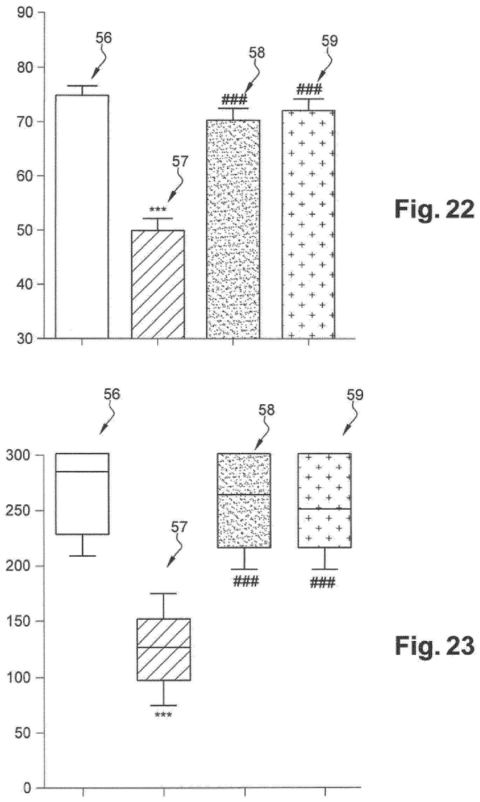

Une partie du groupe de souris auxquelles le peptide amyloïde Aβ25-35 a été injecté a été soumise à un traitement d'irradiation transcutanée du jour 1 (2 heures après l'injection du peptide amyloïde Aβ25-35) au jour 10. Le traitement d'irradiation a été réalisé au niveau de la tête et de l'abdomen. Les dispositifs d'irradiation utilisés sont soit selon l'invention (référence 59 sur les figures 22 à 26), soit en dehors du champ de l'invention (référence 58 sur les figures 22 à 26). Les différents dispositifs utilisés et comparés seront explicités plus loin.

Aux jours 8 à 10, des tests comportementaux sont réalisés sur tous les groupes de souris (Sc.Aβ sans traitement, Aβ25-35 sans traitement, Aβ25-35 avec traitement).

Le premier test comportemental réalisé le jour 8 évalue l'altération de la mémoire spatiale spontanée des souris au moyen d'un test d'évaluation de la performance d'alternance dans un labyrinthe en Y. Le labyrinthe comporte donc trois bras. Chaque souris est positionnée à l'extrémité d'un bras et peut se déplacer librement dans le labyrinthe pendant une session de 8 minutes. Le déplacement de chaque souris y compris les retours dans un même bras sont vérifiés visuellement. Une alternance est définie comme des entrées dans les trois bras à plusieurs occasions consécutives. Le nombre d'alternances maximales est le nombre total d'entrées dans les bras moins deux. Le pourcentage d'alternance est calculé comme étant : (le nombre d'alternances réelles/le nombre d'alternances maximales) x 100. Les résultats de ce premier test comportemental sont présentés sur les figures 10, 16 et 22.

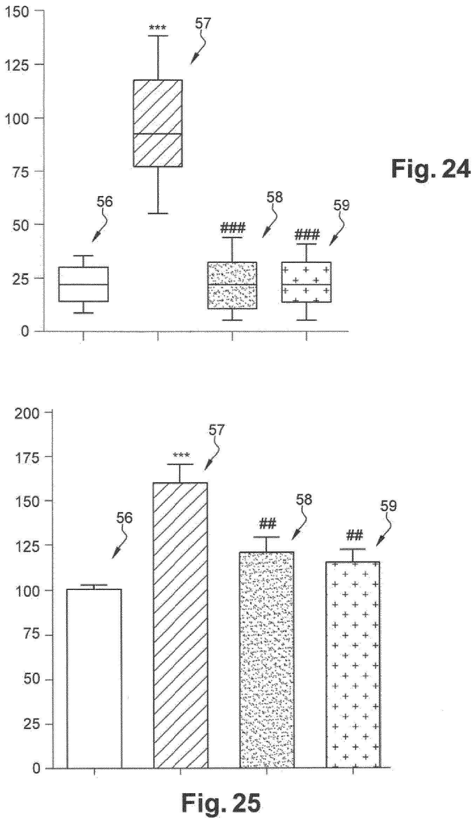

Le second test comportemental réalisé les jours 9 et 10 évalue la mémoire contextuelle à long terme autrement dénommé « test d'évitement passif ». Ce test est réalisé le jour 10 en deux temps avec une séance de formation le jour 9. L'appareil faisant l'objet du test est une boîte à deux compartiments dont l'une est éclairée et l'autre plongée dans le noir et munie d'un plancher sous forme de grille. Une porte à fermeture de type guillotine sépare les deux compartiments. Des chocs peuvent être générés au niveau du planche en grille du compartiment obscur. Initialement, la porte séparant les deux compartiments est fermée. Pour la séance d'entraînement, chaque souris est placée dans le compartiment éclairé. Après 5 secondes, la porte est ouverte. Lorsque la souris pénètre dans le compartiment obscur, on génère des chocs électriques sur la grille. Au jour 10, la souris est de nouveau placée dans le compartiment éclairé, la porte fermée. La porte est ouverte et on mesure deux paramètres : le temps de latence, c'est-à-dire le temps au bout duquel la souris pénètre dans le compartiment obscur, et le temps d'échappement, c'est-à-dire le temps au bout duquel la souris sort du compartiment obscur. Les résultats de ces deux sous-tests (temps de latence et temps d'échappement) sont présentés sur les figures 23 et 25.

Au jour 10, les souris sont euthanasiées. L'hippocampe et le cortex frontal des souris sont disséqués. On détermine les taux de peroxydation lipidique dans l'hippocampe en équivalents CHP par milligrammes de tissu et en pourcentage par rapport au groupe témoin (Sc.Aβ sans traitement). Les résultats sont présentés sur la figure 24. On détermine également dans l'hippocampe par la méthode immuno-enzymatique ELISA le taux de TNFβ (facteur de nécrose tumorale). Les résultats sont exprimés en pourcentage par rapport au groupe témoin (Sc.Aβ sans traitement) et présentés sur la figure 26.

Il est à noter que pour les résultats présentés sur les figures 22 à 26, les résultats obtenus par injection de Sc.Aβ sans traitement constituent un premier témoin de référence puisque cette injection n'a pas modifié le comportement des souris ni le taux de marqueurs testés. Les résultats obtenus par injection de Aβ 25-35 sans traitement constituent un second témoin de référence.

On notera également que les indications ### et ## signifient respectivement une totale et excellente adéquation avec le groupe témoin (Sc.Aβ sans traitement), et les indications *** signifient une totale inadéquation avec le témoin (Sc.Aβ sans traitement).

On se réfère aux figures 22 à 26. Ces figures illustrent les résultats obtenus par les tests précédemment évoqués pour des traitements d'irradiations réalisés une fois par jour sous les conditions opératoires suivantes :
- Référence 56 : injection de Sc.Aβ sans traitement (témoin 1)
- Référence 57 : injection de Aβ25-35 sans traitement (témoin 2)
- Référence 58 : injection de Aβ25-35 avec traitement de 6 minutes une fois par jour simultanément sur la tête et sur l'abdomen par un dispositif d'irradiation A pour lequel les modules d'irradiation comportent une lentille transparente plane appliquée sur la peau des souris préalablement rasées et qui comporte trois diodes électroluminescentes (LED) émettant dans l'infrarouge à 850 nanomètres, trois diodes électroluminescentes (LED) émettant dans le rouge à 640 nanomètres, et une diode laser de type pulsée présentant une durée d'impulsion comprise entre 80 et 100 nanosecondes, un train d'impulsion de 10 kHz (soit 0,1 milliseconde), de préférence entre 1 et 20kHz, typiquement 15 kHz, émettant à 850 nanomètres et présentant une puissance impulsionnelle de 1 Watt. Le traitement est établi en mode pulsé à 10 Hertz pour la tête et pour l'abdomen.
- Référence 59 : injection de Aβ25-35 avec traitement de 6 minutes une fois par jour simultanément sur la tête et sur l'abdomen par un dispositif d'irradiation B pour lequel les modules d'irradiation comportent un guide optique selon l'invention de la quatrième variante illustrée sur les figures 20 et 21, les modules étant appliqués sur la tête et l'abdomen des souris non rasées. Le module comporte une diode électroluminescente (LED) émettant dans l'infrarouge à 850 nanomètres, une diode électroluminescente (LED) émettant dans le rouge à 640 nanomètres, et une diode laser de type pulsé présentant une durée d'impulsion comprise entre 80 et 100 nanosecondes, un train d'impulsion de 10 kHz (soit 0,1 milliseconde), de préférence entre 1 et 20kHz, typiquement 15 kHz, émettant à 850 nanomètres et présentant une puissance impulsionnelle de 1 Watt. Le traitement est établi en mode pulsé à 10 Hertz pour la tête et pour l'abdomen.

On constate que les résultats présentés sur les figures 22 à 26 sont très significatifs pour le dispositif d'irradiation B (référence 59) selon l'invention. On constate également que ces résultats sont équivalents aux résultats obtenus pour le dispositif A de l'art antérieur. Ainsi, le guide optique de l'invention permet d'assurer une bonne efficacité de traitement même en présence de poils ou cheveux sur la peau.

Pour les trois variantes de réalisation précédemment décrites, le guide optique 1, 1a, 30 doit être maintenu en position sur la tête d'un utilisateur au niveau d'une zone bien précise. Pour ce faire, le guide optique 1 est monté sur un module d'irradiation transcutané qui peut être celui des figures 11 et 19 pour la troisième variante du guide optique, un module d'irradiation comportant la quatrième variante du guide optique, ou tout autre module adapté qui assure le maintien coaxial des sources lumineuses en regard proximal des extrémités collectrices des tiges de diffusion. Le module prévoit également des moyens d'alimentation électrique de la carte électronique.

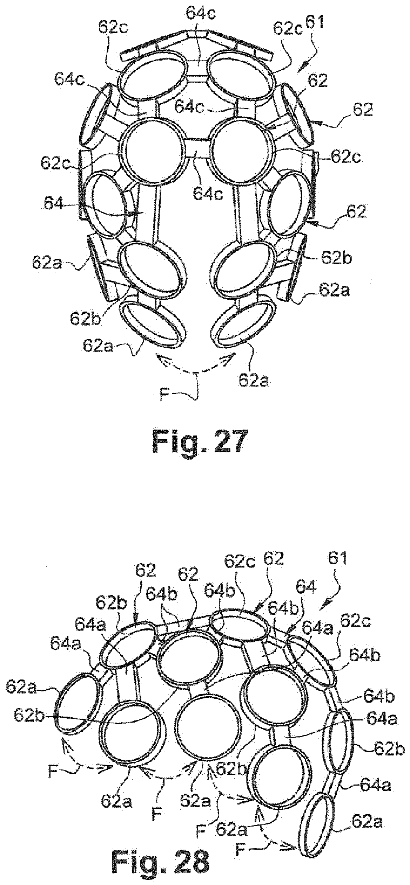

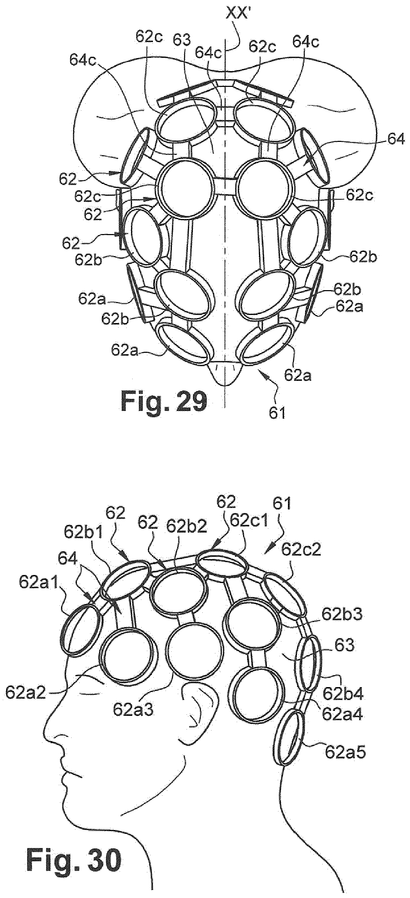

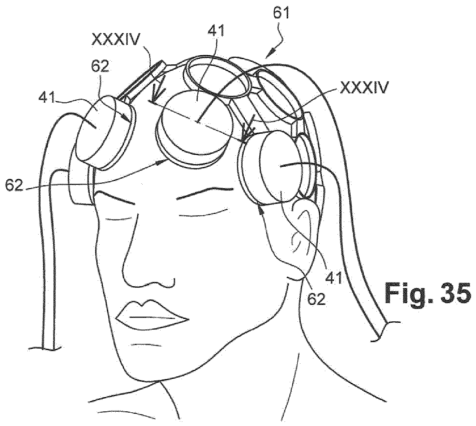

Le module doit être positionné sur la tête de l'utilisateur de façon précise pour irradier uniquement la zone concernée. Pour ce faire, on prévoit un support de modules décrit en référence aux figures 27 à 35.

Le support de modules comporte un ou des anneaux réalisés dans un matériau souple et/ou élastique permettant d'assurer par emprise élastique le maintien fixe du module d'irradiation transcrânienne. On entend par matériau souple et/ou élastique un matériau par exemple élastomère ou en caoutchouc qui autorise l'insertion d'un module de surface externe essentiellement cylindrique dans l'anneau par écartement dudit anneau et retour élastique contre le module. Le matériau pourra être souple sans être élastique, son écartement pour l'insertion et la fixation du module impliquant une sensible extension du matériau, le maintien du module dans l'anneau pouvant alors être assuré par friction. Le support comporte également des moyens de positionnement sur la zone à irradier. Ces moyens peuvent prendre la forme d'une sangle, mais peuvent également, ou en complément, tenir à la forme du support qui, en reposant sur la zone à traiter, s'adaptent à cette zone et sont maintenus en place du fait de cette forme de support adapté.

On se réfère aux figures 27 à 32 pour décrire le support de modules de l'invention.

Le support 61 comporte une pluralité d'anneaux 62 de maintien de modules répartis à la surface de la tête 63 (pour des raisons de clarté, tous les anneaux ne sont pas référencés sur les figures). À titre d'exemple non limitatif, chaque anneau est réalisé en silicone, présente un rayon externe de 25 millimètres, un rayon interne de 23 millimètres (soit une épaisseur de 2 millimètres), et une hauteur de 6 millimètres. La hauteur doit être suffisante à la tenue d'un module qui sera décrit plus loin. En variante de l'utilisation de silicone, les anneaux peuvent être réalisés en caoutchouc, en matériau élastomère ou en tout autre matériau polymérique ou non qui est suffisamment souple pour permettent l'insertion et le maintien d'un module de surface externe essentiellement cylindrique.

Les anneaux 62 sont répartis symétriquement sur le support, de façon que lorsqu'ils sont positionnés sur la tête 63, l'axe de symétrie du support XX' coïncide avec l'axe médian XX' de la tête 63 de l'utilisateur. Les anneaux 62 sont reliés en entre eux par des éléments de jonction souples 64, réalisés par exemple dans le même matériau que les anneaux, dans cet exemple en silicone. Les éléments de jonction 64 sont précisément positionnés sur le support 61 pour permettre à la fois, au support 61 de s'adapter à la forme de la tête 63 de l'utilisateur, et pour permettre le maintien du support sur la tête 63, comme il sera décrit en détail plus loin.

Les anneaux 62 se répartissent de la façon suivante : le support 61 prévoit dix anneaux périphériques 62a, quatre anneaux de seconde périphérie 62b et quatre anneaux centraux 62c. Il est entendu que ce nombre précis et cette disposition précise des anneaux est donnée à titre d'exemple non limitatif. Le nombre d'anneaux 62 peut varier, leur position également, tout en restant dans le cadre de l'invention.

On se réfère à la figure 28 sur laquelle les anneaux 62a,62b,62c ont été sous numérotés 62a1,62a2,62a3,62a4,62a5,62b1,62b2,62b3,62b4,62c1,62c2 pour décrire leur fonctionnalité, étant entendu que cette figure 28 ne représente qu'une moitié du support 61, Les quatre anneaux périphériques 62a1,62a2 et les deux anneaux de seconde périphérie 62b1 situés vers l'avant de la tête 63 sont destinés à atteindre le lobe frontal. Les six anneaux périphériques 62a2,62a3,62a4 s'étendant depuis le second anneau périphérique avant sont destinés à atteindre le lobe temporal. Les quatre anneaux centraux 62c1,62c2 et les quatre anneaux de seconde périphérie 62b2,62b3 situés au-dessus des oreilles du patient sont destinés à atteindre le lobe pariétal. Les quatre anneaux périphériques 62a4,62a5 les plus en arrière sont destinés à atteindre le cervelet. Et les deux anneaux de seconde périphérie 62b4 situés au-dessus de l'anneau périphérique le plus en arrière, et les deux anneaux périphériques 62a4 à l'avant des anneaux périphériques 62a5 les plus en arrière, sont destinés à atteindre le lobe occipital. Tous les anneaux 62 permettent également d'atteindre plus profondément le thalamus, l'hippocampe et les amygdales.

Les quatre anneaux centraux 62c sont reliés entre eux par des jonctions centrales 64a. Les anneaux de seconde périphérie 62b sont reliés aux anneaux centraux 62c par des jonctions de seconde périphérie 64b. Certains anneaux de seconde périphérie 62b sont également reliés entre eux par des jonctions de seconde périphérie 64b. Les anneaux périphériques 62a sont chacun relié à un anneau de second périphérie 62b par une jonction périphérique 64a. En revanche, les anneaux périphériques 62a ne sont pas reliés entre eux par des jonctions. Il est possible de prévoir que l'une des jonctions 64a,64b,64c comporte une zone plate permettant d'y solidariser une étiquette d'identification du patient.

L'absence de liaison entre les anneaux périphériques 62a permet au support 61 de s'adapter à la forme de la tête du patient en s'ouvrant plus ou moins. Restant dans le cadre de l'invention, certains anneaux périphériques 62a pourraient être reliés entre eux tout en conférant cette fonctionnalité d'adaptation. En revanche, si tous les anneaux périphériques 62a sont reliés entre eux, le support 61 ne pourra pas s'adapter à différentes formes de têtes.

Pour ce qui est des anneaux de seconde périphérie 62b, certains sont reliés entre eux pour que le support présente une tenue suffisante pour rester en place sur la tête et maintenir les modules en places dans les anneaux. Le fait que certains anneaux de seconde périphérie 62b ne soient pas reliés entre eux permet également d'entretenir la fonctionnalité d'adaptation du support 61 à toute forme de tête.

La présence ou non et la position des jonctions de seconde périphérie 64b et des jonctions périphériques 64a sont appréciées par l'homme du métier dans un compromis entre la rigidité du support 61 nécessaire pour lui permettre d'être maintenu en position sur la tête du patient et de maintenir le ou les modules, et le caractère d'adaptation du support 61 à toute forme de tête. La présence ou non de ces jonctions peut notamment varier selon le nombre d'anneaux présents sur le support 61 ou encore la rigidité des matériaux employés pour réaliser les anneaux 62 et les jonctions 64.

En tout état de cause, il est essentiel qu'une partie au moins des anneaux périphériques 62a ne soient pas reliés entre eux, et de préférence que tous les anneaux périphériques 62a ne soient pas reliés entre eux. Il apparaît également important pour la rigidité du support qu'au contraire, les anneaux centraux 62c soient reliés entre eux par des jonctions 64a. La liaison intégrale des anneaux centraux 62c dépend du nombre d'anneaux centraux 62c utilisés.

À titre d'exemple, le support 61 pourra être réalisé en une seule pièce par moulage de silicone.

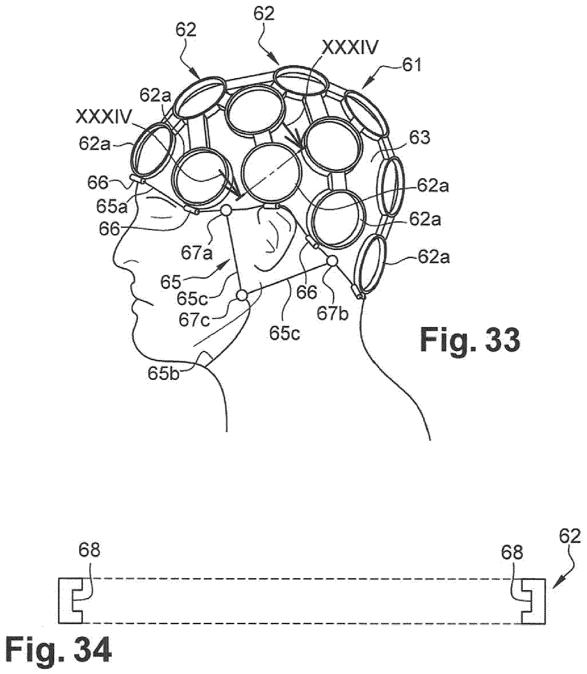

En référence à la figure 33, pour améliorer plus encore le maintien du support 61 sur la tête et pour positionner précisément les anneaux 62 vers les zones du cerveau à traiter, il est prévu une sangle à serrage contrôlé 65. Cette sangle 65 comporte une première partie de liaison 65a qui relie chaque anneau périphérique 62a en passant par un élément tubulaire 66 agencé au niveau du bord inférieur périphérique de chaque anneau périphérique 62a.

La sangle 65 comporte également une mentonnière 65b reliée à la première partie de liaison 65a par quatre éléments de liaison 65c, passant deux à deux de part et d'autre de l'oreille du patient. Alternativement, on peut prévoir que la sangle 65 est maintenue en position par des bandes de type velcro.

On peut prévoir trois points de serrage 67a,67b,67c parmi lesquels deux points de serrage 67a,67b sont situés à la jonction des éléments de liaison 65c et de la première partie de liaison 65a, et le troisième point de serrage est situé à la jonction entre la mentonnière 65b et les deux éléments de liaison 65c.

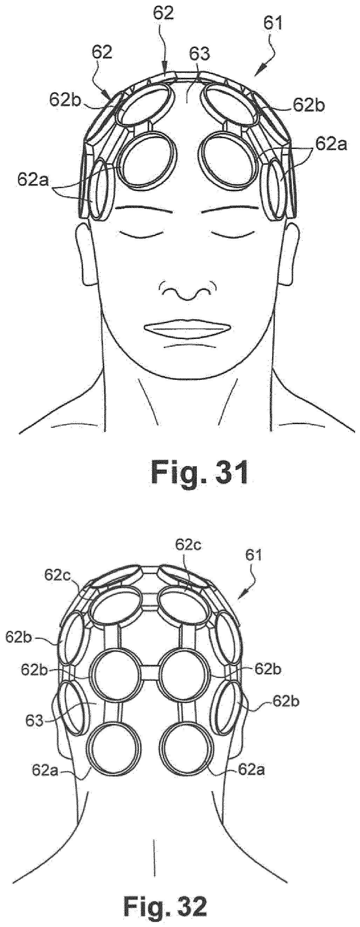

Pour améliorer plus encore la tenue des modules (qui seront décrit plus loin) dans les anneaux 62, on peut prévoir, en référence à la figure 32, la présence d'une rainure 68 au niveau de la face interne de l'anneau 62. Cette rainure 68 est destinée à coïncider avec une nervure circulaire ménagée sur la surface externe du module associé. La présence de la rainure 68 permet non seulement d'améliorer la tenue du module, mais apporte également un positionnement plus précis du module pour que les rayonnements soient précisément dirigés vers les zones ciblées du cerveau.

L'assemblage du module comportant le guide optique de l'invention, par exemple le module 41 de la troisième variante, dans un anneau 62 est réalisé par insertion coaxiale du module 41 à l'intérieur de l'anneau 62. Le module 41 présente à cet effet une forme générale cylindrique permettant d'assurer son maintien coaxial dans l'anneau 62. Cette configuration coaxiale est importante car elle permet d'assurer le positionnement et le maintien fixe du module 41 dans l'axe de l'anneau dédié.

Comme évoqué précédemment, lorsque les anneaux 62 sont pourvus d'une rainure interne 68, la surface externe du module 41 présente une nervure correspondante circulaire venant se loger dans la rainure 68 pour assurer le positionnement fixe et précis du module 41 dans l'axe de l'anneau 62.

Ainsi, la présence du guide optique 1, 1a, 30 sur le module d'irradiation 41, ainsi que le maintien du ou des modules d'irradiation 41 par le support 61 permettent d'irradier précisément et efficacement les zones du cerveau ciblées en augmentant l'efficacité des traitements neurologiques.

Le support de module peut aussi prendre la forme d'un bonnet (ou encore une membrane souple) destiné à être disposé sur la tête d'un patient, bonnet dans lequel des guides optiques sont enfichés par exemple. Également il peut s'agir d'un bonnet dans lequel des orifices sont prévus pour loger les guides optiques.

Le guide optique de l'invention s'applique également à l'irradiation d'autres parties du corps, par exemple l'abdomen de façon indépendante ou concomitante à une irradiation du cerveau.

De manière alternative et en référence à la figure 5a, la tige de diffusion 2' est constituée de deux éléments : un conduit C dans lequel est logé au moins une fibre optique FO qui permet de conduire la lumière depuis une extrémité inférieure 6' jusqu'à une extrémité opposée 7'. La fibre optique FO s'étend longitudinalement à l'intérieure du conduit FO. Le conduit C s'étend longitudinalement depuis la première extrémité inférieure 6' jusqu'à l'extrémité opposée de diffusion 7' destinée à être apposée sur la surface à irradier. Le conduit C présente les mêmes formes que la tige 2 de la figure 5 : partie inférieure 9' présentant un diamètre D1 et partie supérieure 10' de diamètre D2, D2<D1. La tige de diffusion 2' selon ce mode de réalisation peut s'agencer dans la base 3 de la même manière que la tige de diffusion 2.

De même, la forme de la tige de diffusion 2a de la figure 10, peut être celle d'un conduit dans lequel au moins une fibre optique peut être disposée, le conduit peut alors être disposé dans la base 3a de la figure 9 de la même manière que la tige de diffusion 2a.

Également, en considérant une tige de diffusion en deux pièces, on peut prévoir un conduit dans lequel une tige 2, 2a est disposée.

Le conduit est avantageusement en matière plastique et est recouvert, de préférence, à l'intérieur, d'un matériau réfléchissant de manière à favoriser la diffusion de la lumière par la ou les fibre(s) optique(s).

Dans le cas où la tige de diffusion est constituée de deux éléments, il n'est pas nécessaire de prévoir une lentille ou autre à l'extrémité supérieure.

Comme les tiges de diffusion en une seule pièce 2, 2a, la variante en deux pièces avec un conduit peut être un composant d'un module d'irradiation transcutané, dans lequel le guide optique est monté dans un boitier annulaire 4, 4a, 42, la ou les fibre(s) optique(s) étant reliée(s) à une source lumineuse Laser ou infra-rouge. S'agissant du cas avec au moins une fibre optique, la source lumineuse, peut être constituée d'une ou plusieurs sources par exemple Laser qui seront reliées entre elles par une fibre optique.

De manière complémentaire, à toutes les variantes décrites ci-dessus, l'extrémité inférieure de la tige peut prendre plusieurs formes : ronde, triangulaire, carrée, etc. En outre, à cette extrémité, de manière alternative ou complémentaire, un prisme peut être disposé devant la lentille pour améliorer la diffusion de la lumière.

## Revendications

1. Module d'irradiation transcutanée, comprenant un guide optique (1, 1a, 30) de diffusion de rayonnement lumineux à travers une surface, ledit guide optique comprenant une base (3,31) comprenant ou apte à maintenir une pluralité de tiges de diffusion (2, 2a ; 35a,35b,35c) dont chaque tige comporte l'extrémité inférieure de diffusion (7, 7a ; 38a,38b,38c) faisant saillie de ladite base (3) et étant destinée à être appliquée sur ou à proximité de ladite surface, l'extrémité supérieure collectrice (6,6a 37a,37b,37c) de chaque tige étant destinée à être située à proximité et en regard d'une source lumineuse (36a,36b,36c) alimentée électriquement, la tige de diffusion (2, 2a ; 35a,35b,35c) comprenant un matériau apte à transmettre la lumière depuis son extrémité collectrice (6,6a ; 37a,37b,37c) jusqu'à son extrémité de diffusion (7, 7a ; 38a,38b,38c), le module comprenant un boîtier annulaire (4, 4a, 42) comportant une pluralité de sources lumineuses (36a, 36b, 36c) située en regard et à proximité de l'extrémité collectrice (37a, 37b, 37c) de la pluralité de tiges de diffusion (2, 2a, 35a, 35b, 35c), chaque tige de diffusion (2, 2a, 35a, 35b, 35c) étant associée à une source lumineuse de la pluralité de sources lumineuses (36a, 36b, 36c); le guide optique (1, 1a, 30) étant monté dans le boîtier annulaire (4, 4a, 42); la pluralité de sources lumineuses (36a, 36b, 36c) comprenant une diode électroluminescente (36b) émettant dans le spectre rouge, une diode électroluminescente (36a) émettant dans le spectre infrarouge et une diode laser (36c) infrarouge.

2. Module selon la revendication 1, **caractérisé en ce que** la tige de diffusion est d'une pièce.

3. Module selon l'une des revendications précédentes, **caractérisé en ce que** l'extrémité collectrice (37a,37b) de la tige de diffusion (35a,35b) comporte une lentille convergente.

4. Module selon l'une des revendications précédentes, **caractérisé en ce que** l'extrémité inférieure de diffusion (38a,38b,38c) est plane.

5. Module selon l'une des revendications précédentes, **caractérisé en ce que** la tige de diffusion (35a,35b,35c) est réalisée en polyméthacrylate de méthyle (PMMA), ou en verre, ou en polycarbonate ou en copolyester transparent (PETG) ou encore en tout autre matériau équivalent à haute transparence.

6. Module selon la revendication 1, **caractérisé en ce que** la tige de diffusion est de deux pièces et constituée d'un conduit (C) dans lequel au moins une fibre optique (FO) est disposée.

7. Module selon la revendication précédente, **caractérisé en ce que** le conduit est recouvert, à l'intérieur, d'un matériau réfléchissant.

8. Module selon l'une des revendications précédentes, **caractérisé en ce que** la base (3,31) est réalisée en un matériau apte à transmettre la lumière.

9. Module selon l'une des revendications précédentes, **caractérisé en ce que** la base (3) comporte une pluralité d'orifices traversant (15) à travers chacun desquels est logée une tige de diffusion amovible (2,2a).

10. Module selon l'une quelconque des revendications 1 à 6, **caractérisé en ce que** le guide optique est réalisé en une seule pièce intégrant la ou les tiges de diffusion (35a,35b,35c).

11. Module selon l'une des revendications précédentes, **caractérisé en ce que** l'extrémité de diffusion (7, 7a ; 38a,38b,38c) comporte une lentille convergente et/ou un prisme.

12. Module d'irradiation selon l'une des revendications précédentes, **caractérisé en ce que** la diode laser infrarouge est de type pulsé.

13. Module d'irradiation selon la revendication 12, **caractérisé en ce que** la diode laser de type pulsé émet dans l'infrarouge à une longueur d'onde comprise entre 700 et 1200 nanomètres, présente une durée d'impulsion comprise entre 20 et 200 nanosecondes, un train d'impulsion compris entre 1 et 10 kHz et une puissance impulsionnelle comprise entre 0,5 et 12 Watts, de préférence entre 1 et 20kHz, typiquement 15 kHz.

14. Module d'irradiation selon l'une quelconque des revendications précédentes, **caractérisé en ce que** la fréquence de modulation appliquée aux diodes électroluminescentes et au laser infrarouge est comprise entre 1 et 1000 Hz.

## Patentansprüche

1. Modul zur transkutanen Bestrahlung, umfassend eine optische Führung (1, 1a, 30) zur Diffusion von Lichtstrahlung durch eine Oberfläche, wobei die optische Führung eine Basis (3, 31) umfasst, die eine Vielzahl von Diffusionsstäben (2, 2a; 35a, 35b, 35c) umfasst oder zu halten imstande ist, wobei jeder Stab das untere Diffusionsende (7, 7a; 38a, 38b, 38c) aufweist, das aus der Basis (3) hervorsteht und bestimmt ist, auf der Oberfläche oder in deren Nähe angewendet zu werden, wobei das obere Sammelende (6, 6a; 37a, 37b, 37c) jedes Stabs bestimmt ist, sich in der Nähe und einer elektrisch versorgten Lichtquelle (36a, 36b, 36c) zugewandt zu befinden, wobei der Diffusionsstab (2, 2a; 35a, 35b, 35c) ein Material umfasst, das imstande ist, das Licht von seinem Sammelende (6, 6a; 37a, 37b, 37c) bis zu seinem Diffusionsende (7, 7a; 38a, 38b, 38c) zu übertragen, wobei das Modul ein ringförmiges Gehäuse (4, 4a, 42) umfasst, das eine Vielzahl von Lichtquellen (36a, 36b, 36c) aufweist, die sich in der Nähe und dem Sammelende (37a, 37b, 37c) der Vielzahl von Diffusionsstäben (2, 2a, 35a, 35b, 35c) zugewandt befindet, wobei jeder Diffusionsstab (2, 2a, 35a, 35b, 35c) einer Lichtquelle von der Vielzahl von Lichtquellen (36a, 36b, 36c) zugeordnet ist; wobei die optische Führung (1, 1a, 30) in dem ringförmigen Gehäuse (4, 4a, 42) angebracht ist; wobei die Vielzahl von Lichtquellen (36a, 36b, 36c) eine Leuchtdiode (36b), die im roten Spektrum sendet, eine Leuchtdiode (36a), die im Infrarot-Spektrum sendet und eine Infrarot-Laserdiode (36c) umfasst.

2. Modul nach Anspruch 1, **dadurch gekennzeichnet, dass** der Diffusionsstab aus einem Teil ist.

3. Modul nach einem der vorangehenden Ansprüche, **dadurch gekennzeichnet, dass** das Sammelende (37a, 37b) des Diffusionsstabs (35a, 35b) eine konvergierende Linse aufweist.

4. Modul nach einem der vorangehenden Ansprüche, **dadurch gekennzeichnet, dass** das untere Diffusionsende (38a, 38b, 38c) eben ist.

5. Modul nach einem der vorangehenden Ansprüche, **dadurch gekennzeichnet, dass** der Diffusionsstab (35a, 35b, 35c) aus Polymethylmethacrylat (PMMA) oder aus Glas oder aus Polycarbonat oder aus transparentem Copolyester (PETG) oder auch aus jedem anderen äquivalenten hochtransparenten Material hergestellt ist.

6. Modul nach Anspruch 1, **dadurch gekennzeichnet, dass** der Diffusionsstab aus zwei Teilen ist und aus einer Leitung (C) besteht, in der mindestens eine optische Faser (FO) angeordnet ist.

7. Modul nach vorangehendem Anspruch, **dadurch gekennzeichnet, dass** die Leitung innen mit einem reflektierenden Material bedeckt ist.

8. Modul nach einem der vorangehenden Ansprüche, **dadurch gekennzeichnet, dass** die Basis (3, 31) aus einem Material hergestellt ist, das imstande ist, das Licht zu übertragen.

9. Modul nach einem der vorangehenden Ansprüche, **dadurch gekennzeichnet, dass** die Basis (3) eine Vielzahl von Durchgangsöffnungen (15) aufweist, durch die hindurch jeweils ein lösbarer Diffusionsstab (2, 2a) untergebracht ist.

10. Modul nach einem der Ansprüche 1 bis 6, **dadurch gekennzeichnet, dass** die optische Führung aus einem einzigen Teil hergestellt ist, das den oder die Diffusionsstäbe (35a, 35b, 35c) integriert.

11. Modul nach einem der vorangehenden Ansprüche, **dadurch gekennzeichnet, dass** das Diffusionsende (7, 7a; 38a, 38b, 38c) eine konvergierende Linse und/oder ein Prisma aufweist.

12. Bestrahlungsmodul nach einem der vorangehenden Ansprüche, **dadurch gekennzeichnet, dass** die Infrarot-Laserdiode vom gepulsten Typ ist.

13. Bestrahlungsmodul nach Anspruch 12, **dadurch gekennzeichnet, dass** gepulste Laserdiode im Infrarot in einer Wellenlänge sendet, die zwischen 700 und 1200 Nanometer liegt, eine Impulsdauer zwischen 20 und 200 Nanosekunden, eine Impulsfolge zwischen 1 und 10 kHz und eine Impulsleistung zwischen 0,5 und 12 Watt, vorzugsweise zwischen 1 und 20 kHz, in typischer Weise von 15 kHz, aufweist.

14. Bestrahlungsmodul nach einem der vorangehenden Ansprüche, **dadurch gekennzeichnet, dass** die auf die Leuchtdioden und auf den Infrarotlaser angewendete Frequenzmodulation zwischen 1 und 1000 Hz liegt.

## Claims

1. A transcutaneous irradiation module, comprising an optical light guide (1, 1a, 30) for diffusing a light radiation through a surface, said optical light guide comprising a base (3, 31) comprising or being configured to hold a plurality of diffusing rods (2, 2a; 35a, 35b, 35c), whose each rod has the lower diffusing end (7, 7a; 38a, 38b, 38c) protruding from said base (3) and being intended to be applied on or near said surface, the upper collecting end (6, 6a; 37a, 37b, 37c) of each diffusing rod being intended to be located in the vicinity of and facing an electrically powered light source (36a,36b,36c), the diffusing rod (2,2a; 35a,35b,35c) comprising a material capable of transmitting light from its collecting end (6,6a; 37a,37b,37c) to its upper end (6,6a; 37a,37b,37c), the module comprising an annular housing (4, 4a; 42), comprising a plurality of light sources (36a, 36b, 36c) located opposite and close to the collecting end (37a, 37b, 37c) of the plurality of diffusing rods (2, 2a; 35a, 35b, 35c), , each diffusing rode being associated to a light source of the plurality of light sources (36a, 36b, 36c); the optical guide (1, 1a, 30) being mounted in the annular housing (4, 4a, 42); the plurality of light sources (36a, 36b, 36c) comprising a light emitting diode (36b) emitting in the red spectrum, a light emitting diode (36a) emitting in the infrared spectrum and an infrared laser diode (36c).

2. The module of claim 1, **characterized in that** the diffusing rod is in one piece.

3. Module according to any of the preceding claims, **characterized in that** the collecting end (37a, 37b) of the diffusing rod (35a, 35b) comprises a converging lens.

4. Module according to one of the preceding claims, **characterized in that** the lower diffusion end (38a, 38b, 38c) is planar.

5. Module according to one of the preceding claims, **characterized in that** the diffusing rod (35a, 35b, 35c) is made of polymethyl methacrylate (PMMA), or of glass, or of polycarbonate or of transparent copolyester (PETG) or of any other equivalent material with high transparency.

6. Module according to claim 1, **characterized in that** the diffusing rod is in two-piece and consists of a conduit (C) in which at least one optical fiber (FO) is arranged.

7. A module according to the preceding claim, **characterized in that** the duct is coated on the inside with a reflective material.

8. A module according to any of the preceding claims, **characterized in that** the base (3, 31) is made of a material configured tof transmit light.

9. Module according to one of the preceding claims, **characterized in that** the base (3) comprises a plurality of through holes (15) through each of which a removable diffusing rod (2, 2a) is housed.

10. Module according to any one of claims 1 to 6, **characterized in that** the optical guide is made in one piece integrating the diffusion rod or rods (35a, 35b, 35c).

11. Module according to any of the preceding claims, **characterized in that** the diffusion end (7, 7a; 38a, 38b, 38c) comprises a converging lens and/or a prism.

12. Irradiation module according to any of the preceding claims, **characterized in that** the infrared laser diode is of the pulsed type.

13. Irradiation module according to claim 12, **characterized in that** the pulsed type laser diode emits in the infrared at a wavelength between 700 and 1200 nanometers, has a pulse duration between 20 and 200 nanoseconds, a pulse train between 1 and 10 kHz and a pulse power between 0.5 and 12 Watts, preferably between 1 and 20kHz, typically 15 kHz.

14. Irradiation module according to any of the preceding claims, **characterized in that** the modulation frequency applied to the light-emitting diodes and the infrared laser is between 1 and 1000 Hz.
